# EUROPÄISCHE PATENTSCHRIFT

(11) **EP 1 156 830 B1**
(45) Veröffentlichungstag und Bekanntmachung des Hinweises auf die Patenterteilung: **11.01.2006**
(21) Anmeldenummer: 00914043.5
(22) Anmeldetag: 23.02.2000
(51) Int. Cl.: A61K 47/24, A61K 31/695

(54) **VERWENDUNG EINER ÖL-IN-WASSER-EMULSION ZUR HERSTELLUNG EINES ARZNEIMITTELS**
USE OF AN OIL-IN-WATER EMULSION FOR THE MANUFACTURE OF A MEDICAMENT
UTILISATION D'UNE EMULSION HUILE DANS EAU DESTINÉE À L'OBTENTION D'UN MÉDICAMENT

(30) Priorität: 24.02.1999 DE 19910161
(43) Veröffentlichungstag der Anmeldung: 28.11.2001
(73) Patentinhaber: Exner, Heinrich, 39291 Möckern (DE); Klose, Peter, 39291 Grabow (DE)
(72) Erfinder: Exner, Heinrich, 39291 Möckern (DE); Klose, Peter, 39291 Grabow (DE)
(74) Vertreter: Kietzmann, Manfred
(86) Internationale Anmeldenummer: PCT/DE2000/000559
(87) Internationale Veröffentlichungsnummer: WO 2000/050085

(56) Entgegenhaltungen:
- DE-A- 4 445 074
- DE-A- 4 499 552

## Beschreibung

Die Erfindung betrifft die Verwendung einer Silikonölemulsion zur Herstellung eines Medikamentes.

### [Stand der Technik]

Im Patent DE 44 99 552 ist ein Adjuvans für Antigene und ein Verfahren zu seiner Herstellung und Verwendung unter Schutz gestellt. In dieser Patentschrift ist ein inkomplettes Adjuvans in Form einer Ö1-in-Wasser-Emulsion beschrieben, bestehend aus
- 0,01-30 % Polydimethylsiloxanen, die einen Polymerisationsgrad von n = 20-400 und eine kinematische Viskosität von 20-1000 mm²/s bei 20°C aufweisen,
- 0,01-15 % Komplexemulgator mit einem HLB-Wert von 9 -16,
- 45-99 % biokompatibler Salzlösung,
- 0,01-10 % Dimethylsulfoxid und
- 0, 0001-1 % Chelatbildner.

Diesem inkompletten Adjuvans können zur Immunisierung Peptidoglykane auf der Basis von speziesspezifischen St.aureus-Stämmen und/oder anderen Stämmen in einer Konzentration von 0,00001 bis 1 mg Eiweißstickstoff pro ml Adjuvans und wasserlösliche natürliche und/oder synthetische Polymere in einer Konzentration von 0,0001 bis 10 mg pro ml Adjuvans zugesetzt werden.

Mit dieser Erfindung werden Adjuvantien zur Verfügung gestellt, die durch die Kombination mit Impfantigenen oder in Kombination mit Peptidoglycanen in einer histokompatiblen Formulierung die Abwehrmechanismen im Körper so weit stimulieren, daß neben der aktiven Immunprophylaxe auch schwacher Antigene eine allgemeine und spezifische Immuntherapie erfolgt. Mit diesem Adjuvans werden gute Erfolge bei Immuntherapien erreicht. Von Nachteil ist, daß die Herstellung des kompletten Adjuvans bzw. das Hinzufügen von Proteinen relativ aufwendig ist. Ein pathophysiologischer Nachteil besteht in der Gefahr der anaphylaktischen Reaktion des Organismus bei mehrmaliger Applikation des kompletten Adjuvans.

Im Patent DE 44 99 552 ist auch die Verwendung des inkompletten Adjuvans für die Herstellung von Vakzinen und für die Immunisierung in der Human- und Veterinärmedizin beschrieben. Das Patent erhält jedoch keine brauchbaren Informationen für die alleinige Anwendung des inkompletten Adjuvans als Arzneimittel für konkrete Krankheiten. Bei allen in diesem Patent angeführten Beispielen wird eine Vakzination durchgeführt, bei der die Patienten durch das Impfen mit einer geschwächten Form eines Organismus gegen einen Infektionsorganismus immun gemacht werden bzw. geheilt werden sollen.

### [Aufgabe der Erfindung]

Der Erfindung liegt die Aufgabe zugrunde, die Verwendung einer Öl-in-Wasser-Emulsion zur parenteralen, insbesondere intramuskulären und/oder subkutanen Applikation vorzuschlagen, die frei von Proteinen ist und die allein oder in Kombination mit anderen intramuskulär und/oder subkutan applizierbaren Wirkstoffen zur Herstellung eines Medikamentes zur Behandlung oder Prophylaxe von Multiple Sklerose, Morbus Crohn, Colitis ulcerosa, chronischen Erschöpfungszuständen, verwendet wird.

Die vorliegende Erfindung beruht auf der Feststellung, daß die Öl-in-Wasser-Emulsion in der Zusammensetzung
- 0,01-30 % Polydimethylsiloxanen, die einen Polymerisationsgrad von n = 20-400 und eine kinematische Viskosität von 20-1300 mm2/s aufweisen,
- 0,01-15 % hydrophiler Emulgator,
- 45-99 % biokompatibler Salzlösung,
- 0,01-10 % Dimethylsulfoxid

zur Herstellung eines Medikamentes zur Behandlung oder Prophylaxe von Morbus Crohn, Colitis ulcerosa, Erschöpfungszuständen, Multiple Sklerose verwendet werden kann.

Die Ö1-in-Wasser-Emulsion kann zusätzlich wenigstens einen der Bestandteile 0,0001-1 % Chelatbildner und Glycerol in einer Konzentration von 0,01 - 10 % enthalten.

Der hydrophile Emulgator kann aus 25-35 % Sorbitantrioleat, 25-35 % Cetylstearylalkohol und 35-45 w/w% Polysorbat 80 bestehen.

Es wurde nämlich festgestellt, daß die Öl-in-Wasser-Emulsion in der oben aufgeführten Zusammensetzung bei parenteraler, insbesondere intramuskulärer und/oder subkutaner Applikation überraschend gute Wirkungen bei den oben genannten Indikationen zeigt. Durch die Anwendung dieser Emulsion wird ein nachhaltiger Effekt erzielt. Bei der Behandlung mit dem erfindungsgemäßen Medikament ist die Dauer der Behandlung bis zum ersten Effekt und die gesamte Dauer der Behandlung gegenüber der Behandlung mit herkömmlichen Medikamenten deutlich verkürzt. Die Behandlungsdauer und die Dauer bis zum Eintritt eines ersten Effektes läßt sich durch die Wiederholung der Applikation innerhalb weniger Stunden und über mehrere Tage hinweg weiter verkürzen. Mit der Behandlung verbundene belastende Nebenwirkungen sind nicht beobachtet worden.

Die Applikation des erfindungsgemäßen Medikamentes führt auch bei solchen Indikationen zu guten Ergebnissen, die als unheilbar gelten oder bei denen der Krankheitsverlauf mit herkömmlichen Medikamenten lediglich verzögert werden kann. Das gilt insbesondere für Multiple Sklerose. Bei Patienten, bei denen die Applikation herkömmlicher Medikamente weder Heilung noch Linderung brachte und die als austherapiert galten, ist durch die Applikation des erfindungsgemäßen Medikamentes eine deutliche Verbesserung der Gesundheit und des Gesamtbefindens eingetreten.

Weiterhin hat sich gezeigt, dass die Kombination der erfindungsgemäßen Emulsion mit anderen parenteral applizierbaren Wirkstoffen zu überraschenden Effekten führt. Wird die erfindungsgemäße Emulsion mit wenigstens einem Wirkstoff aus den Wirkstoffgruppen Antibiotika, Kortikoide, Antiparkinsonmittel, Mittel gegen Multiple Sklerose, Neuro- und Psychopharmaka, Immunstimulantien und Schmerzmittel kombiniert, dann reduziert sich die Medikation dieser. Wirkstoffe gegenüber der üblichen Medikation ohne Kombination mit der erfindungsgemäßen Emulsion um bis zu 50% und in Einzelfällen um mehr als 60%. Auf diese Weise lassen sich bei voller oder gesteigerter Wirksamkeit dieser Wirkstoffe deren Nebenwirkungen auf ein Minimum reduzieren.

### [Beispiele]

### Beispiel 1

Die Öl-in-Wasser-Emulsion wird in der Zusammensetzung
- 0,01-30 % Polydimethylsiloxanen, die einen Polymerisations-grad von n = 20-400 und eine kinematische Viskosität von 20-1300 mm²/s aufweisen,
- 0,01-15 % hydrophiler Emulgator,
- 45-99 % biokompatibler Salzlösung,
- 0,01-10 % Dimethylsulfoxid

gebildet.

Die Öl-in-Wasser-Emulsion kann zusätzlich wenigstens einen der Bestandteile 0,0001-1 % Chelatbildner und 0,01 - 10 % Glycerol enthalten.

Der hydrophile Emulgator kann aus 25-35 % Sorbitantrioleat, 25-35 % Cetylstearylalkohol und 35-45 % Polysorbat 80 bestehen.

Zusätzlich zu den oben genannten Inhaltsstoffen kann der Emulsion 0,02 g/ml Glycerol zugegeben werden.

Als besonders wirksam hat sich eine erfindungsgemäße Emulsion mit folgenden Inhaltsstoffen und Massenanteilen (g/ml Emulsion) erwiesen:
- 0,01-0,03 Polydimethylsiloxane, die einen Polymerisationsgrad von n = 20-400 und eine kinematische Viskosität von 20-1300 mm²/s aufweisen;
- 0,01-0,03, Dimethylsulfoxid,
- 0,03-0,08 hydrophiler Emulgator,
- 0,86-0,95 isotonische Kochsalzlösung.

Zusätzlich zu den genannten Inhaltsstoffen kann die Emulsion EDTA Na/Ca Salz in einem Massenanteil von 0,0001-0,01 g/ml enthalten.

Die Bestandteile der Emulsion werden nach den bekannten emulsionschemischen Regeln zusammengeführt. Die Emulsion ist sterilfiltrierbar, autoklavierbar und kann eingefroren werden. Wird die Emulsion durch eine dieser Maßnahmen gespalten, kann sie anschließend wieder reemulgiert werden.

Es hat sich gezeigt, daß die erfindungsgemäße Öl-in-Wasser-Emulsion besonders gute Ergebnisse zeigt bei der Verwendung als Medikament zur Behandlung von Multiple Sklerose, Morbus Crohn, Colitis ulcerosa, chronischen Erschöpfungszuständen bzw. zur Herstellung von Mitteln zu deren Bekämpfung.

### Beispiel 2

Bei der Diagnose eine der unter Beispiel 1 genannten Erkrankungen beginnt die Therapie mit einem drei- bis siebentägigen (mitunter auch bis 14tägigen) Applikationsintervall, bis eine Stabilisierung der Gesundheit und der Leukozyten im Normalbereich erreicht ist. Neben der Stabilisierung der Leukozyten sollte auch die CD 4, CD 8, NK, TNF alpha Werte erfaßt werden. In Abhängigkeit von der Schwere der jeweiligen Erkrankung und des Alters des Patienten werden die Normwerte im Durchschnitt nach zwei- bis vierwöchiger Behandlung erreicht.

Nach dem drei- bis siebentägigen Applikationsintervall werden bis zur klinischen Stabilisierung die Applikationen im Abstand von 7 - 14 Tagen vorgenommen.

Ist eine klinische Stabilisierung erreicht, d.h. hält eine gewisse klinische Genesung stabil über 4 bis 6 Wochen an, kann der Applikationsintervall auf etwa 14 Tage ausgedehnt werden.

Zur Initialisierung des Heilungsprozesses kann auch eine mehrmalige Applikation dieser Emulsion im Abstand von wenigen Stunden innerhalb eines Tages erfolgen.

Die Dauer der Restitution der geschädigten Gewebe erfordert eine Behandlungszeit von mindestens 6 bis 12 Monaten. Bei Erkrankungen des Knochensystems kann dieser Wert noch überschritten werden.

Bei chronischen Verlaufsformen wie z.B. MS müssen die Therapien bei einem Applikationsintervall von etwa zwei bis drei Wochen ständig erhalten bleiben, damit diese Krankheiten nicht wieder exazerbieren.

### Beispiel 3

### Multiple Sklerose (MS)

### Status präsens:

Die Patientin, 50 Jahre alt und Invalidenrentnerin leidet an MS. Die Krankheit MS ist bei dieser Patientin seit 10 Jahren gesichert. Der Verlauf ging mit zunehmender Lähmung des linken Armes und des linken Beines einher. Erste Anzeichen beobachteten sie bereits seit ihrem zweiten Lebensjahrzehnt. Auch ein zunehmender Sehverlust des rechten Auges war zu beobachtet. Alle bisherigen Behandlungen (Cortison, Interferon, usw.) haben die kontinuierliche Verschlechterung nicht aufhalten können. Die Patientin konnte sich seit einem Jahr nicht mehr allein versorgen. Alternative Behandlungen haben der Patientin nicht geholfen. Es wurden alle Therapien abgebrochen und die Patientin wartete auf das Ende des Lebens.

### Behandlung:

Die Behandlung mit der erfindungsgemäßen Emulsion erfolgte im wöchentlichen Abstand. Dazu erhielt die Patientin je 1 ml Emulsion i.m. verabreicht. Die Patientin wurde während der Behandlung durch den Augenarzt, Hausarzt und Neurologen kontrolliert (klinische und Laboruntersuchungen).

Nach 14 Tagen begann die Patientin bereits zu registrieren, dass sie etwas mehr Kraft im erkrankten Bein und im erkrankten Arm hatte. Sie konnte besser schlafen und die Zuckungen im Bein waren nur noch selten zu beobachten.

Nach 6-wöchiger Behandlung ging die auf die Inaktivität zurückzuführende Blaufärbung am linken Arm zurück. Die Patientin stolperte weniger und die Nervenschwäche des linken Beines (Peroneus) wurde merklich weniger. Sie konnte sich ab diesem Zeitpunkt stärker belasten.

Nach 10-wöchiger Behandlung konnte die Patientin mit Unterstützung einer Begleitperson bereits einen Kilometer gehen (vor der Behandlung konnte sie maximal 200 Meter bewältigen). Die Schwierigkeiten bei der Nahrungsaufnahme (Verschlucken) verschwanden.

Nach 14-wöchiger Behandlung wurde eine deutliche Verbesserung des Allgemeinbefindens und der Beweglichkeit festgestellt: Die Hand konnte bereits wieder über den Kopf gehoben werden; die Kraft der Hand war deutlich gestiegen; die Patientin konnte ihre täglichen häuslichen Aufgaben allein bewältigen; ihr Gang war sicherer geworden; sie hatte keine Kopfschmerzen mehr und sie war frei von Krämpfen.

Der klinische Verlauf der MS der Patientin wurde gestoppt und innerhalb des Behandlungsjahrs wurde sie durch die Behandlung mit der erfindungsgemäßen Emulsion soweit physisch und psychisch restituiert, so dass sie sich jetzt als nahezu gesund bezeichnet. Die Gehstrecke hat sich auf 2 km erhöht und der Augenarzt der Patientin konnte die Aufhebung der Sehnervschwäche diagnostizieren.

Bei einer größeren Operation wegen einer Materialentfernung am rechten Unterschenkel ist ein dreitägiger Spritzrhythmus vorgenommen worden. Diese Operation wurde folgenfrei überstanden. Es kam weder zu einer MS Verschlechterung noch zu einem Schub.

Injektionen werden weiterhin im Abstand von zwei Wochen durchgeführt.

### Beispiel 4

### Morbus Crohn

### Status präsens:

Der Patient, 54 Jahre alt, 176 m groß, Gewicht 65 kg, Haut blass, Tonus nachlassend, starke Knochen- und Muskelschmerzen, keine Belastbarkeit, Gebiss saniert, aber Lockerung aller Zähne, Haare grau, geistig müde, teilweise depressive Stimmungslage, enteritische Symptome mit Rectumbefall, Schlafstörungen, Infektanfälligkeit.

### Behandlung:

Im Abstand von zwei bis drei Wochen eine Injektion mit 1 ml der erfindungsgemäßen Emulsion.

Nach 4 bis.6 Wochen gingen die enteritischen Symptome zurück, die vorher zu beobachtende Gewichtsreduktion stagnierte und zeigte bei gleicher Ernährung einen leichten Aufwärtstrend. Die Muskel- und Knochenschmerzen gingen zurück.

Nach einem Vierteljahr waren die Muskel- und Knochenschmerzen rheumatoiden Charakters, die seit dem 10. Lebensjahr mit unterschiedlicher Intensität präsent waren, im Wesentlichen zurückgegangen. Die Schlafstörungen hörten auf, es folgten längere erholsame Schlafpausen.

Nach einem halben Jahr war das Ausgangsgewicht vor dem Status präsens mit 85 kg wieder erreicht. Schultergürtel und Oberarmbereich entwickelten sich mit starker Muskelmasse. Die Zähne wurden wieder fest im Halteapparat. Die Haare an den Augenbrauen und auf dem Kopf wurden systematisch durch Haare der ursprünglichen Dicken ersetzt. Erste Beobachtungen zum Rückgang der grauen Haare und zum stärkeren Wachstum von Haaren und Nägeln folgten.

Nach einem Jahr übersteigt die Leistungsfähigkeit bei weitem den Stand zu Beginn des 5. Lebensjahrzehnts. Die grauen Haare wurden ersetzt durch pigmenthaltige, im Farbton etwas dunkler als die ursprüngliche Behaarung. Ein Stagnieren im Alterungs-Prozess ist eingetreten. Alle Organsysteme haben ihre volle Funktionsfähigkeit wieder erhalten.

Eine deutliche Verbesserung der Nutzung der Nährstoffe im Organismus macht eine Gewichtskontrolle im Rahmen dieser Therapie notwendig.

Die Redoxsysteme des Organismus wurden ebenfalls regeneriert. So ist nach dieser Therapie beispielsweise die Toleranz gegenüber direkter Sonnenstrahlung erheblich vergrößert worden. Der typisch penetrante Schweißgeruch war nicht mehr vorhanden. Die Haut wurde straff, elastisch und von gesunder Farbe.

Die Behandlung wird mit Injektionen im Abstand von zwei bis drei Wochen weitergeführt.

### Beispiel 5

### Colitis ulcerosa Migräne

### Status präsens:

Die Patientin, 47 Jahre, leidet an Colitis ulcerosa und an Migräne.

### Behandlung:

Die Injektionen mit der erfindungsgemäßen Emulsion erfolgten in den ersten 4 Wochen im Abstand von 4 Tagen, danach für 5 Monate im wöchentlichen und anschließend im zweiwöchigen Abstand. Dazu erhielt die Patientin je 0,5 ml Emulsion i.m. verabreicht. Ein erster Effekt war nach 3 Wochen zu erkennen. Innerhalb der Behandlung über 16 Monate ist kein Rezidiv eingetreten.

### Beispiel 6

### Chronisches

### Erschöpfungssyndrom (CFS)

### Status präsens:

Der Patient, 61, leidet am chronischen Erschöpfungssyndrom.

### Behandlung:

Die Injektionen mit der erfindungsgemäßen Emulsion erfolgten in den ersten 3 Monaten im wöchentlichen Abstand, danach für 4 Monate im zweiwöchentlichen Abstand und seit dem im Abstand von 2 - 3 Wochen. Dazu erhielt der Patient je 0,5 ml Emulsion i.m. verabreicht. Ein erster Effekt war nach 8 Wochen zu erkennen. Der Patient fühlt sich seit diesem Zeitpunkt völlig gesund. Die Behandlung wurde zur Rezidivvorbeugung über 9 Monate fortgesetzt.

## Patentansprüche

1. Verwendung einer Öl-in-Wasser-Emulsion, bestehend aus
a) 0,01-30 % Polydimethylsiloxanen, die einen Polymerisationsgrad von n = 20-400 und eine kinematische Viskosität von 20-1300 mm²/s aufweisen,
b) 0, 01-15 % hydrophiler Emulgator,
c) 45-99 % biokompatibler Salzlösung,
d) 0,01-10 % Dimethylsulfoxid,
zur Herstellung eines Medikamentes zur Behandlung von chronischen Erschöpfungszuständen, Colitis ulcerosa, Morbus Crohn, Multiple Sklerose.

2. Verwendung einer Öl-in-Wasser-Emulsion nach Anspruch 1, **dadurch gekennzeichnet, daß** die Emulsion 0,0001-1 % Chelatbildner enthält.

3. Verwendung einer Öl-in-Wasser-Emulsion nach einem der Ansprüche 1 und 2, **dadurch gekennzeichnet, dass** der hydrophile Emulgator aus
• 25-35% Sorbitantrioleat,
• 25-35% Cetylstearylalkohol,
• 35-45% Polysorbat 80.

4. Verwendung einer Ö1-in-Wasser-Emulsion nach einem der Ansprüche 1 bis 3, **dadurch gekennzeichnet, dass** die Emulsion Glycerol in einer Konzentration von 0,01 - 10 % enthält.

5. Verwendung einer Öl-in-Wasser-Emulsion nach Anspruch 1, **dadurch gekennzeichnet, dass** die Emulsion aus folgenden Stoffen mit den folgenden Massenanteilen in g/ml gebildet wird:
• 0,01-0,03 Polydimethylsiloxane, die einen Polymerisationsgrad von n = 20-400 und eine kinematische Viskosität von 20-1300 mm²/s aufweisen,
• 0,01-0,03 Dimethylsulfoxid,
• 0,03-0,08 hydrophiler Emulgator,
• 0,86-0,95 isotonische Kochsalzlösung.

6. Verwendung einer Öl-in-Wasser-Emulsion nach Anspruch 5, **dadurch gekennzeichnet, dass** die Emulsion EDTA Na/Ca Salz in einem Massenanteil von 0,0001-0,01 g/ml enthält.

7. Verwendung einer Öl-in-Wasser-Emulsion nach einem der Ansprüche 1 bis 6, **dadurch gekennzeichnet, dass** die Emulsion mit wenigstens einem der folgenden parenteral applizierbaren Wirkstoffe kombiniert wird: Antibiotika, Kortikoide, Antiparkinsonmittel, Mittel gegen Multiple Sklerose, Neuro- und Psychopharmaka, Immunstimulantien, Schmerzmittel.

## Revendications

1. Utilisation d'une émulsion huile dans eau composée de
a) 0,01 à 30 % de polydiméthylsiloxanes présentant un degré de polymérisation n = 20 à 400 et une viscosité cinématique de 20 à 1300 mm²/s,
b) 0,01 à 15 % d'émulsifiant hydrophile,
c) 45 à 99 % de solution saline biocompatible,
d) 0,01 à 10 % de diméthylsulfoxyde,
pour la fabrication d'un médicament destiné au traitement d'états d'épuisement chronique, colite ulcéreuse, maladie de Crohn, sclérose multiple.

2. Utilisation d'une émulsion huile dans eau selon la revendication 1, **caractérisée en ce que** l'émulsion contient de 0,0001 à 1 % d'agents chélateurs.

3. Utilisation d'une émulsion huile dans eau selon l'une des revendications 1 et 2, **caractérisée en ce que** l'émulsifiant hydrophile est composé de
• 25 à 35 % de sorbitane trioléate,
• 25 à 35 % d'alcool cétyle-stéaryle,
• 35 à 45 % de polysorbate 80.

4. Utilisation d'une émulsion huile dans eau selon l'une des revendications 1 à 3, **caractérisée en ce que** l'émulsion contient du glycérol dans une concentration de 0,01 à 10 %.

5. Utilisation d'une émulsion huile dans eau selon la revendication 1, **caractérisée en ce que** l'émulsion est formée des substances suivantes avec les parts en poids suivantes, en g/ml :
• 0,01 à 0,03 de polydiméthylsiloxanes présentant un degré de polymérisation n = 20 à 400 et une viscosité cinématique de 20 à 1300 mm²/s,
• 0,01 à 0,03 de diméthylsulfoxyde,
• 0,03 à 0,08 d'émulsifiant hydrophile,
• 0,86 à 0,95 de solution saline isotonique.

6. Utilisation d'une émulsion huile dans eau selon la revendication 5, **caractérisée en ce que** l'émulsion contient du sel EDTA-Na/Ca dans une part en poids de 0.0001 à 0.01 g/ml.

7. Utilisation d'une émulsion huile dans eau selon l'une des revendications 1 à 6, **caractérisée en ce que** l'émulsion est combinée à l'une des substances actives parentéralement administrables suivantes : antibiotiques, corticoïdes, médicaments anti-Parkinson, médicaments contre la sclérose multiple, substances neurotropes et psychotropes, immunostimulants, analgésiques.

## Claims

1. The use of an oil-in-water emulsion consisting of
a) 0.01-30% polydimethylsiloxanes having a degree of polymerisation of n = 20-400 and a kinematic viscosity of 20-1,300 mm²/s,
b) 0.01-15% hydrophilic emulsifier,
c) 45-99% biocompatible salt solution,
d) 0.01-10% dimethyl sulphoxide,
for the manufacture of a medicinal drug for treating chronic fatigue conditions, colitis ulcerosa, Crohn's disease, multiple sclerosis.

2. The use of an oil-in-water emulsion according to Claim 1, **characterized in that** said emulsion contains 0.0001-1% chelating agents.

3. The use of an oil-in-water emulsion according to any one of Claims 1 and 2, **characterized in that** said hydrophilic emulsifier consists of
- 25-35% sorbitan trioleate,
- 25-35% cetylstearyl alcohol,
- 35-45% Polysorbate 80.

4. The use of an oil-in-water emulsion according to any one of Claims 1 to 3, **characterized in that** said emulsion contains glycerol in a concentration ranging between 0.01 and 10%.

5. The use of an oil-in-water emulsion according to Claim 1, **characterized in that** said emulsion is composed of the following substances with the following masses per unit volume in g/ml:
- 0.01-0.03 polydimethylsiloxanes having a degree of polymerisation of n = 20-400 and a kinematic viscosity of 20-1,300 mm²/s,
- 0.01-0.03 dimethyl sulphoxide,
- 0.03-0.08 hydrophilic emulsifier,
- 0.86-0.95 isotonic sodium chloride solution.

6. The use of an oil-in-water emulsion according to Claim 5, **characterized in that** said emulsion contains EDTA Na/Ca salt with a mass per unit volume ranging between 0.0001 and 0.01 g/ml.

7. The use of an oil-in-water emulsion according to any one of Claims 1 to 6, **characterized in that** said emulsion is combined with at least one of the following, parenterally applicable active agents:
antibiotics, corticoids, antiparkinson agents, agents against multiple sclerosis, neuropharmacological and psychopharmacological agents, immune stimulants, analgesics.
